# EUROPEAN PATENT SPECIFICATION

(11) **EP 1 218 746 B1**
(45) Date of publication and mention of the grant of the patent: **21.12.2005**
(21) Application number: 00964517.7
(22) Date of filing: 05.10.2000
(51) Int. Cl.: G01N 33/558, G01N 33/543

(54) **FLUID-FLOW CONTROL DEVICE**
FLÜSSIGKEITSSTRÖMUNGSSTEUERVORRICHTUNG
DISPOSITIF DE CONTROLE D'ECOULEMENT DE FLUIDE

(30) Priority: 05.10.1999 GB 9923384
(43) Date of publication of application: 03.07.2002
(73) Proprietor: THE UNIVERSITY OF BIRMINGHAM, Edgbaston, Birmingham B15 2TT (GB)
(72) Inventor: BUNCE, Roger, Abraham, Birmingham B30 1DZ (GB); FREITAG, Helmut, Birmingham B30 1DX (GB)
(74) Representative: Ward, David Ian
(86) International application number: PCT/GB2000/003825
(87) International publication number: WO 2001/025789

(56) References cited:
- EP-A- 0 590 695
- EP-A- 0 596 867
- WO-A-93/03176
- WO-A-96/24060
- WO-A-99/27364
- US-A- 4 522 923
- US-A- 5 354 692

## Description

The present invention relates in a first aspect to a liquid-flow control device.

There are a large number of assay devices for determining chemical or biochemical analytes in a sample. One common type of device comprises a strip of porous material impregnated in at least one discrete region with a reagent sensitive to the analyte of interest. The reagent is usually chosen so that the presence of the analyte is readily observable (eg. appearance or change in colour or fluorescence). A sample is applied to one region of the strip and liquid (eg. water) is applied to the same or another region. As an alternative, the sample may be the liquid or contained in the liquid. The liquid is drawn by capillary action along the strip and carries the sample through the region(s) containing the reagent(s), until saturation of the strip occurs when liquid reaches the far end of the strip. Any analyte of interest present in the sample will contact the reagent(s) as the liquid passes along the strip. Such devices may include built-in procedural controls and/or end of test indicators which are well known in the art.

One disadvantage of such devices is that the contact period between sample and reagent is dependent upon the rate of flow of the sample across the region(s) containing the reagent(s). For certain analyte/reagent pairs, this period may not be long enough for sufficient reaction to occur, i.e. such a device lacks the necessary sensitivity to measure the presence of certain analytes.

US 5874216 discloses a device having a chromatographic strip and a separate sample preparation region on opposable components which can be brought into opposition. The sample preparation region may incorporate a reagent with which the analyte of interest binds and after an appropriate reaction time the opposable components are brought together so that the sample contacts the chromatographic strip and flows into a detection zone.

A successful assay may be dependent upon the user allowing the correct time to elapse between sample preparation and application of sample to the chromatographic strip.

US 4727019 discloses a device which partly overcomes the above disadvantage. The device comprises a porous membrane in contact with an absorbent member. The porous membrane is impregnated with a receptor for a ligand of interest. Sample containing the ligand of interest is applied to the upper side of the membrane and is drawn through the membrane into the absorbent member by capillary action. A labelled receptor which also binds the ligand of interest is then applied to the porous membrane to react with the receptor-ligand complex so that the receptor-ligand complex may be observed. Sensitivity of the reaction may be controlled by applying a large amount of sample to the membrane and/or allowing a longer time period between applying the sample and the labelled receptor.

However, disadvantages are (i) the use of such a device may require large amounts of sample, (ii) the sample and labelled ligand are applied to the membrane manually and (iii) the period between application of the sample and ligand must be measured.

None of the above prior art devices are able to control the assay by controlling the flow of the liquid along the porous medium.

W096/24060 discloses an assay device and method for detecting the presence of an analyte in a sample involving sequential delivery of a labelled binding reagent and a label developing means to a detection site. Sequential delivery is achieved by differential channel lengths in a liquidic circuit or the use of slow release compositions, certain semi permeable barriers or hydrophobic surfactants to control hydrophobicity of the membrane.

EP0596867 discloses a method for determination of a component of a sample using monoclonal antibodies. The required sequential delivery of components of the assay to a detection site is controlled by the use of soluble barriers or barriers which are permeable to liquid flow in one direction only.

W099/27364 discloses a one-step enzyme immunoassay in which enzyme-antibody conjugate or label and enzyme substrate are separated until separation of bound and free enzyme conjugate or label is complete. This separation is accomplished by, inter alia, using variable flow paths or liposomes in which the substrate is imbibed, the liposome being carried in the flow path to a desired location before being ruptured.

US4,522,923 describes an apparatus and method for conducting immunochemical reactions in which at least three chambers are separated by impermeable barriers. In one embodiment, the chambers are stacked vertically in a test tube. The barriers can be designed to be ruptured by suitably alkaline liquids.

Thus, an object of the present invention is to provide a liquid-flow control device capable of use as an assay device eg. for determining chemical or biochemical analytes present in a sample. Another object is to provide a device which obviates or mitigates one or more disadvantages of the prior art assay devices.

According to a first aspect of the present invention, there is provided a liquid-flow control device comprising:-
(i) a liquid flow path, defined by interstices of a porous medium
(ii) a liquid flow barrier at a location in the flow path, the liquid flow barrier being a non-wetting region in the flow path, and
(iii) barrier release means impregnated in the porous medium in the flow path, the barrier release means being a wetting agent capable of wetting said liquid flow barrier and which, in use, is moveable by liquid flowing in the flow path into contact with the fluid flow barrier so as to permit liquid flow through said location.

It will be understood that there is no flow through said location until the barrier is contacted in use by the barrier release means.

Preferably, said liquid is an aqueous liquid.

Preferably, the liquid flow barrier is a hydrophobic material and the release means is a surfactant. Examples of suitable hydrophobic materials include waxes (e.g. paraffin wax), resins (e.g. acrylic resin based varnish, 100% acrylic polymer varnish, coumarone-indene resin) and commercially available synthetic polymers, inks and paints. Examples of suitable surfactants include octyl-β-D-glucopyranoside (ODG), dioctyl sulfosuccinate sodium salt (DOSS), polyoxyethylene (23) dodecyl ether (BRIJ 35) and polyoxyethylene (20) sorbitan monolaurate (TWEEN 20).

The flow path is defined by interstices (i.e. capillaries and/or pores) of a porous medium. Preferably said porous medium is a nitrocellulose-based material.

Preferably, the liquid flow barrier is impregnated into and immobilised on the porous medium. Preferably, the barrier release means is soluble in the flowing fluid.

Preferably, the device is constructed so that, in use, there is at least one region in the flow path where the direction of liquid flow is different after liquid flow through said location is initiated compared with the direction of liquid flow in that region before liquid flow through said location is initiated. More preferably the liquid flow direction is reversed.

The present invention also resides in an assay device for an analyte comprising
(i) a flow path for a liquid defined by interstices in a porous medium,
(ii) at least one liquid flow barrier which is impregnated into and immobilised on the porous medium at a location in the flow path, said barrier being a non-wetting region in the flow path
(iii) at least one barrier release means which is a metting agent capable of wetting said liquid flow barrier, is impregnated into the porous medium, is soluble in the liquid, and which, in use, is moveable by liquid flowing in the flow path into contact with the flow barrier so as to permit liquid flow through said location, and
(iv) an analyte capture region in the flow path.

Preferably, an analyte visualising agent is provided at a zone in the flow path, said visualising agent being capable of interacting with the analyte to indicate the presence of the analyte. Alternatively, the device may be provided with a visualising agent application zone adapted to receive a visualising agent.

As used herein, the expression "visualising agent" encompasses any agent which interacts with the analyte directly or indirectly via an intermediary analyte binding reagent (eg. enzyme labelled conjugate) and which enables the visual or instrumental detection of analyte qualitatively, semi-quantitatively or quantitatively eg. by colour, fluorescence, luminescence or radioactivity. It will be understood that the visualising agent may be inherently detectable, or detectable once interaction with the analyte or intermediary analyte binding reagent has occurred.

In a first embodiment, the visualising agent is immobilised in the capture region and also serves to immobilise the analyte in the capture region.

In a second embodiment, the analyte capture region includes an immobilised analyte binding substance which serves to immobilise analyte in the capture region and the visualising agent is upstream of the capture region so that in use, it flows through the capture region.

The device may include a sample application zone in the flow path which may, if present, correspond to the analyte capture region or the visualising agent application zone. The sample application zone may be surrounded by an additional flow barrier and an associated barrier release means. Alternatively, the sample to be analysed may be included in the liquid which flows, in use, along the flow path.

In a third embodiment, at least two barriers and at least two barrier release means are provided and arranged so that, in use, some liquid flows over the analyte capture region at least twice.

In a fourth embodiment, a flow path geometry is selected which causes, in use, a predetermined difference in fluid flow within the analyte capture region so that distribution of analyte within the analyte capture region is non-uniform in a predictable manner, whereby the amount of analyte can be determined in at least a semi-quantitative manner.

In those embodiments where more than one barrier is present, the barriers may have different constitutions to each other. Similarly where more than one barrier release means is present, the barrier release means may have different constitutions to each other.

Embodiments of the invention will now be described by way of Example only, with reference to the accompanying drawings, in which:-
Figure 1 show a flow control device in accordance with the present invention used to test various barriers and surfactants,
Figures 2a, 2b and 2c show assay devices in accordance with the present invention for incubating a sample and reagent at a capture site (Figure 2a), or for incubating a sample and reagent before flowing over a capture site (Figure 2b), or for improving the uniformity of switching barriers in devices of the type shown in Figures 2a and 2b.
Figure 3 shows an assay device in accordance with the present invention for causing repeated sample flow across a capture site,
Figure 4 shows an assay device in accordance with the present invention for first delivering a large sample volume through a capture site, followed by analyte labelling,
Figure 5 shows an assay device in accordance with the present invention for isolating a sample region from its surroundings,
Figure 6a shows a membrane strip for use in an assay device in accordance with the present invention where a relatively large volume of sample moves vertically through the thickness of the strip and horizontally along the strip,
Figure 6b is a sectional view of the assay device incorporating the strip of Figure 6a,
Figure 7a shows an assay device in accordance with the present invention where a relatively large volume of sample moves vertically through the thickness of a membrane and is directed by a coacting wicking member to cause horizontal flow along said membrane,
Figure 7b is a cross section through the device at Figure 7a, and
Figures 7c to 7e show the sequence of assembly of the device of Figures 7a and 7b.

In the following Examples, the use of "up", down, "left" and "right" and other reference directions relates to the orientations shown in the drawings.

### EXAMPLE 1 Porous media, barrier and surfactant interaction

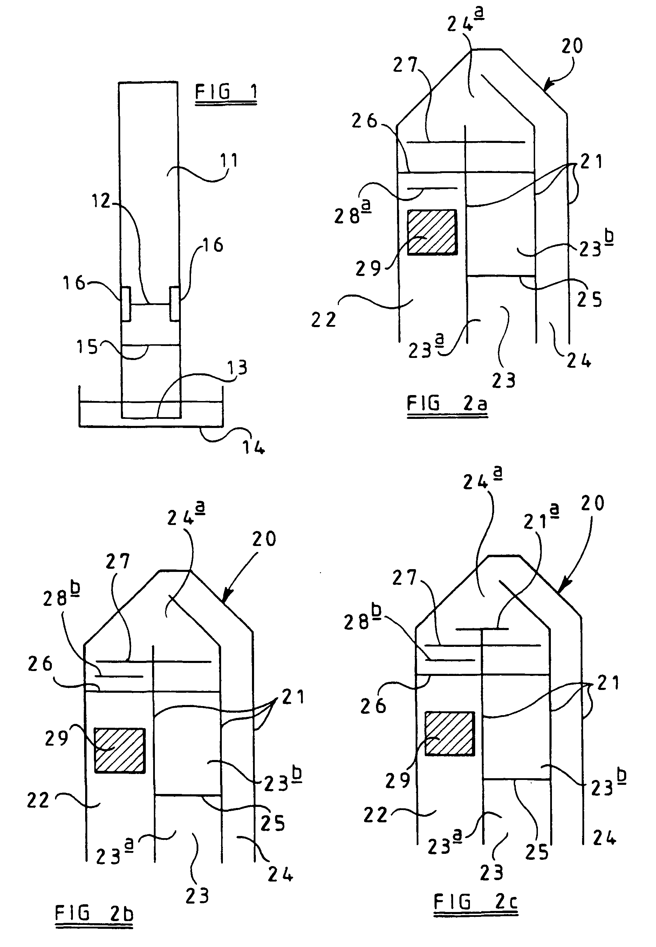

Referring to Figure 1 a polyester supported nitrocellulose membrane sheet of 3 µm or 8 µm nominal pore size (Whatman International Ltd, Maidstone England) was cut into a 10 mm wide by 60 mm long strip 11 with a barrier 12, 20 mm from one end 13. The barrier 12 was applied to the sheet using a Type MP5200 flat bed plotter (Graphtec Corporation, Yokohama, Japan) prior to cutting. A short perpendicular line 16 was drawn manually at each end of the barrier 12 along the cut edges of the strip 11 using a Lumocolor pen. The strip 11 was dried for 10 minutes at 35°C in an air circulating over before use. Several such strips 11 were prepared from each sheet and strips were stored in sealed containers prior to use.

In use, the end 13 of the strip was dipped vertically into about a 3 mm depth of liquid (water or surfactant-containing water, the surfactant serving as a barrier release means), in a vessel 14. Liquid wicked up the membrane to the barrier 12 and the time taken for the liquid to penetrate ("switch") the barrier 12 was noted. The pen lines 16 prevented liquid circumventing the barrier and wicking up the cut edges of the strip 11. To see the passage of liquid more clearly a blue line 15 was manually drawn just below the barrier using a water soluble 0.6 mm "Note Writer" pen (Berol, Banford, Norfolk,UK).

The flow characteristics of different combinations of membrane, barrier and surfactant were investigated. Tables 1 to 11 show for each combination (i) the maximum permissible concentration (%) of surfactant in the liquid (water) to delay barrier switching by at least ten minutes (max conc. %), (ii) the concentration (%) of surfactant required to allow rapid switching of the barrier (switching conc. %), and (iii) the transition time (switching time) across the barrier for liquid at the same concentration as (ii), defined as the time in seconds from liquid touching the underside of the barrier to when it starts to appear on the opposite side of the barrier (in the absence of barrier the transition time is < 5s).

**Table 1 -**

| **Yellow Pen Barrier**^{**1**} **(8 µm membrane)** | | | | |
|---|---|---|---|---|
| | ODG² | DOSS³ | BRIJ 35⁴ | TWEEN 20⁵ |
| max conc. % | 0.03 | 0.015 | 0.5 | 0.5 |
| switching conc. % | 0.125 | 0.06 | 2.5 | 5 |
| Transition time (s) | 16 | 14 | 60 | 43 |

| | | | | |
|---|---|---|---|---|
| ¹Paint Marker Type 751 (Edding AG, Ahrensburg, Germany) | | | | |
| ²ODG (Octyl-β-D-glucopyranoside) (Fluka, Buchs, Switzerland). | | | | |
| ³DOSS (Dioctyl sulfosuccinate, sodium salt) (Sigma-Aldrich Co Ltd, Poole, UK). | | | | |
| ⁴BRIJ 35 (Polyoxyethylene (23) dodecyl ether) (ICN Biolchemicals, Cleveland, USA). | | | | |
| ⁵TWEEN 20 (Polyoxyethylene(20)sorbitan monolaurate) (Sigma-Aldrich Co Ltd.) | | | | |

**Table 2 -**

| **Green Pen Barrier**^{**1**} **(8 µm membrane)** | | | | |
|---|---|---|---|---|
| | ODG | DOSS | BRIJ 35 | TWEEN 20 |
| max conc. % | 0.03 | 0.015 | 0.5 | 0.5 |
| switching conc. % | 0.25 | 0.06 | 2.5 | 5 |
| Transition time (s) | 16 | 14 | 60² | 43 |

| | | | | |
|---|---|---|---|---|
| ¹Paint Marker Type 780 (Edding AG) fitted with a tip from pen type 751. | | | | |
| ²5% BRIJ 35 has the same transition time but flows more slowly thereafter | | | | |

**Table 3 -**

| **White Pen Barrier**^{**1**} **(8 µm membrane)** | | |
|---|---|---|
| | ODG | DOSS |
| max conc. % | 0.125 | 0.03 |
| switching conc. % | 1.25 | 0.125 |
| Transition time (s) | 11 | 26 |

| | | |
|---|---|---|
| ¹Paint Marker Type 751 (5% TWEEN and 5% BRIJ showed slow uneven switching.) | | |

**Table 4 -**

| **Silver Pen Barrier**^{**1**} **(8 µm membrane)** | |
|---|---|
| | ODG |
| max conc. % | 0.125 |
| switching conc. % | 1.25 |
| Transition time (seconds) | 10 |

| | |
|---|---|
| ¹Paint Marker Type 751 (5% TWEEN 20, 5% BRIJ and 1 % DOSS did not penetrate the silver barrier.) | |

**Table 5 -**

| **Yellow Pen Barrier (3 µm membrane)** | | | |
|---|---|---|---|
| | ODG | DOSS | BRIJ 35 |
| max conc. % | 0.06 | 0.03 | 1.25 |
| switching conc. % | 2.5 | 1.0 | 5 |
| Transition time (s) | 9 | 45 | 45 |
| (2.5% TWEEN 20 gave slow and uneven switching) | | | |

**Table 6 -**

| **Green Pen Barrier (3 µm membrane)** | | | |
|---|---|---|---|
| | ODG | DOSS | BRIJ 35 |
| max conc. % | 0.06 | Not Done | 1.25 |
| switching conc. % | 2.5 | 1.0 | 5 |
| Transition time (s) | 11 | 60 | 45 |
| (2.5% TWEEN 20 showed slow and uneven switching) | | | |

**Table 7-**

| **White Pen Barrier (3 µm membrane)** | |
|---|---|
| | ODG |
| max conc. % | 0.125 |
| switching conc. % | 2.5 |
| Transition time (s) | 12 |
| (5% TWEEN, 5% BRIJ and 1 % DOSS showed slow & uneven switching) | |

Tables 1 to 7: Plotting speed, 1 cm/s.

**Table 8 -**

| **Lumocolor**^{**1**}**diluted 1 in 4 with 1-propanol (8 µm membrane)** | | |
|---|---|---|
| | ODG | ODG |
| max conc. % | 0.125 | 0.125 |
| switching conc. % | 2.5 | 1.25 |
| Transition time (s) | 5 | 60 |

| | | |
|---|---|---|
| ¹Staedtler, Nuremberg, Germany | | |

**Table 9 -**

| **Lumocolor diluted 1 in 2 with 1-propanol (8 µm membrane)** | | |
|---|---|---|
| | ODG | ODG |
| max conc. % | 0.125 | 0.125 |
| switching conc. % | 2.5 | 1.25 |
| Transition time (s) | 5 | 180 |

Tables 8 and 9: Plotting speed, 5 cm/s, pen size, 0.5 mm.

**Table 10 -**

| **Coumarone resin**^{**1**} **(8 µm membrane)** | | | |
|---|---|---|---|
| Barrier line width (mm) | % ODG | | % TWEEN 20 |
| | 1.25 | 0.062 | 0.5 |
| 1.4² | 5 | >600 | 9 |
| 3.8³ | 7 | >600 | 24 |
| 4.8⁴ | 13 | >600 | 111 |
| | Transition time (s) | | |

| | | | |
|---|---|---|---|
| ¹Prepared by dissolving: 1 g poly coumarone-co-indene resin, Mn 1090 (Sigma-Aldrich) with 4 g dioxane to give a 20% wt/wt stock solution. The working solution was 0.5 ml of stock solution to which was added 3x0.5 ml heptane mixing between each addition (resultant solution: 5.27% resin wt/vol solvents). Barriers were dried at 35°C prior to testing with surfactants. | | | |
| ²single line plotted with 0.5 mm pen at 5 cm/s | | | |
| ³single line plotted with 0.5 mm pen at 1 cm/s | | | |
| ⁴three lines, 1 mm apart, plotted with 0.5 mm pen at 1 cm/s | | | |

Table 12 shows the distance wicked for the wax barrier-ODG surfactant combinations shown in Table 11.

**Table 11-**

| **Wax Barrier (8 µm membrane) - Transition time** | | | | |
|---|---|---|---|---|
| % ODG | % Wax¹ in white spirit | | | |
| | 1.25 | 2.5 | 5 | 10 |
| 1.25 | ^{∼}125 | ^{∼}55 | ^{∼}92 | ^{∼}71 |
| 2.5 | 17 | ^{∼}24 | 11 | ^{∼}12 |
| 5 | 19 | 20 | 8 | 29 |
| 10 | 33 | 21 | 19 | ^{∼}76 |
| | Transition time (s)² | | | |

| | | | | |
|---|---|---|---|---|
| ¹Paraffin Wax mp 51-53°C (BDH, Poole UK) solubilised in white spirit (BDH) by heating gently on a hot plate set at 50°C. | | | | |
| ²Using 8 µm membrane, all wax barriers, from 1.25 to 10% concentration prevented the flow of deionised water for at least 10 minutes (duration of test). Wax barriers below 0.75% wax allow passage of water. 10% TWEEN 20, 10% BRIJ 35, and 1% DOSS did not penetrate the 1.25% or 10% wax. | | | | |
| "^{∼}" = Mean time because the transition was not even along the wax line. | | | | |

Table 12 shows the distance wicked for the wax barrier/ODG surfactant combinations shown in Table 11.

**Table 12-**

| **Wax Barrier (8 µm membrane)- Distance wicked** | | | | |
|---|---|---|---|---|
| % ODG | % Wax | | | |
| | 1.25 | 2.5 | 5 | 10 |
| 1.25 | 28 | 36 | 35 | 31 |
| 2.5 | 38 | 49 | 51² | 37 |
| 5 | 33 | 43 | 47 | 30 |
| 10 | 26 | 35 | 38 | 26 |
| | Wicking distance (mm) 5 minutes after immersion¹ | | | |

| | | | | |
|---|---|---|---|---|
| ¹Without a wax barrier the wicking distance of water was 71 mm | | | | |
| ²5% wax, 2.5% ODG is the optimum combination in respect of rapid switching and distance wicked | | | | |

Tables 11 and 12: Plotting speed 10 cm/s, pen size 0.5 mm.

**Cryla Soluble Gloss Varnish** (Daler Rowney, Berkshire, UK) (an acrylic resin based varnish): Using the 8 µm membrane a barrier of 0.1 % varnish in white spirit (plotted at 10cm/s using a 0.7 mm pen) held back water for at least 10 minutes. A line of 2.5% ODG was applied manually (0.7 pen) about 5 mm below the Cryla line and water applied to the end of the strip. The barrier switched in about 12 seconds but flow through the barrier was uneven compared with other barrier materials.

**Liquitex High Gloss Varnish** (Brinney & Smith Inc., Easton, USA) (100% acrylic polymer varnish): Using the 8 µm membrane a barrier of 80% varnish in water (plotted at 10 cm/s using a 0.7 mm pen) held back water for at least 10 minutes. A line of 2.5% ODG was plotted about 5 mm below the varnish (5 cm/s, 0.7 mm pen) and water applied to the end of the strip. The barrier switched immediately. 70% varnish is marginal as a water barrier because of slight water absorption. This is seen as a bloom on the surface of the varnish after it was applied to an impervious surface, dried, dipped into water for 30 minutes, dried and examined. However, switching was much faster than any of the other barriers and, using an appropriate application method, could be a useful barrier.

**Wax and 3µm membrane** Wax is difficult to use as a barrier on the 3 µm membrane because wax at a concentration of 0.3% allows water to pass through, but at a concentration of 0.6%, water will not pass through the barrier even at 5% ODG. Thus, the useful working range is very small and the wax content of the barrier critical.

### Discussion:

It will be seen that some combinations of porous media, barrier and surfactant result in faster switching of the barrier than others, e.g. 5s for Lumocolor barrier and 2.5% ODG compared with 17s for 1.25% wax barrier and ODG at the same concentration on the same membrane (Table 8 cf. Table 11). For some combinations, switching occurs at a relatively low concentration of surfactant, e.g. 0.06% DOSS (see Table 1) and some surfactants are ineffective in breaking through some barriers, e.g. TWEEN, BRIJ and DOSS through wax barrier (note 2 to Table 11). From the above data, it is clear that a suitable combination can be chosen for the particular analytical system of interest. For example, if it were necessary to have a first surfactant present as part of the analytical reaction(s) but desirable that this surfactant should not cause switching of the barrier, an appropriate choice may be wax for the barrier and TWEEN 20, BRIJ or DOSS for the first surfactant. switching of the barrier could then be controlled by a second surfactant such as ODG.

The flow control devices of Example 1 do not necessarily reflect the performance of practical assay devices, but are intended to illustrate the difference in behaviour of various barrier/surfactant/membrane combinations. In a preferred assay device the surfactant is dried into an area on the membrane during manufacture. In use, the concentration of surfactant at the barrier is dependent upon the concentration of surfactant applied, its volume, geometry, and the geometry and size of the surrounding structure. Additionally, the switching time is not merely dependent upon the nature of the barrier, surfactant and membrane materials, it is also affected by the degree of saturation in the area of surfactant adjacent to the barrier (saturation is the amount of liquid present per unit area of the membrane). This in turn is dependent upon the geometry and size of the device. This saturation effect can be put to important use to form a feedback-like mechanism so that, for example, a barrier may open only when the device is substantially saturated.

The following Examples show how the principles described above can be incorporated into practical assay devices for determining, for example, chemical or biochemical analytes present in samples such as water, urine and blood. It will be understood that the specific reagents used will depend upon the specific analyte(s) of interest.

### EXAMPLES 2a and 2b

Referring to Figure 2a, an assay device comprises a generally rectangular strip of polyester backed 8 µm nitrocellulose membrane 20. Permanent impervious lines 21 formed by the application of neat Lumocolor ink using the pen plotter divide the membrane into first, second and third channels 22,23,24 which extend from the lower end of the device toward its upper end. The second channel 23 is further divided into a lower portion 23a and an upper portion 23b by a horizontal impervious line 25. At their upper end, the first and second channels 22,23 merge into an enlarged common region 24a which separates the upper end of the third channel 24 from the upper ends of the first and second channels 22,23. A switchable barrier 26 (silver paint from pen type 8700 (Edding AG, plotted using a 0.7 mm pen at 1cm/s, and dried at 22° C for 2 minutes) extends across the full width of the first channel 22 and the upper portion 23b of the second channel 23. Spaced above the switchable barrier 26, a line of surfactant 27 (2.5% ODG plotted using a 0.7 pen at 3 cm/s, and dried at 35° C for 20 minutes) extends substantially across the width of the first and second channels 22,23. Spaced below the barrier 26, a capture line 28a comprising an antibody to the analyte of interest immobilised onto the membrane 20, extends substantially across the width of the first channel 22. Spaced below the capture line 28a, also in the first channel 22, is a region 29 containing gold conjugate for labelling the analyte of interest if present in a sample. A solution of methylcellulose is applied to the membrane prior to the addition of the gold conjugate to prevent unspecific binding to the membrane.

In this embodiment, the analyte is one which can form an immunoassay complex such as a protein, eg. for diagnosis of human chorion gonadotropin (hCG), leutinising hormone (hLH) or C-reactive protein (CRP).

In use, the lower end of the device is placed into liquid so that liquid flow ("wicking") is initiated in the first, second and third channels 22,23,24. The liquid may serve as a diluent for a sample to be analysed or it may be the undiluted sample (e.g. urine). In the first channel 22, liquid wicks up to the gold conjugate containing region 29 where the analyte of interest reacts (if present) to form a gold-analyte complex. Liquid flow continues and the gold-analyte complex (as well as any unreacted gold conjugate) is moved by the liquid flow over the capture line 28a until flow is stopped by the barrier 26. The gold-analyte complex is immobilised at the capture line 28a by binding to the antibodies at the capture line 28a. Liquid also flows in the lower portion 23a of the second channel 23, but is prevented from entering the upper portion 23b of the second channel 23 by the impervious line 25 therebetween.

While liquid is stationary in the first channel 22 at the barrier 26 and in the second channel 23 at the line 25, liquid continues to flow into the third channel 24 and down into the common region 24a. The liquid front solubilises the surfactant line 27 and when the liquid reaches the upper side of the switchable barrier 26, the surfactant switches the barrier 26 i.e. converts the capillary structure of barrier 26 from non-wetting to wetting enabling liquid to flow therethrough.

The geometry of the device is such that downward flow from the common region 24a into the first and second channels 22,23 is substantially symmetrical, so that the barrier 26 is switched substantially simultaneously in the first and second channels 22,23. Liquid is rapidly drawn into the dry upper portion 23b of the second channel 23 from the common region 24a which in turn draws liquid from the first channel 22 through the common region 24a into the upper portion 23b of the second channel 23. Hence, any unbound material in channel 22 flows quickly into region 24a, further flow acting to wash the capture line 28a. A small volume of liquid also flows from the third channel 24, through region 24a, into the upper portion 23b of the second channel 23. If the analyte of interest is present in a predetermined amount it will be seen as a coloured line at the capture line 28a. Liquid continues to flow in the first and third channels 22 and 24 until the upper portion 23b of the second channel 23 is saturated or the device is removed from the liquid.

Referring to Figure 2b, a modified assay device is shown which is identical to that shown in Figure 2a except that the capture line 28b is disposed in the first channel 22 between the barrier 26 and the surfactant line 27. The modified device is particularly useful when the reaction time between analyte and visualising agent (in this embodiment gold-conjugate) is critical, prior to their flow over the capture line 28b.

In use, about 10 µl of sample is applied onto the gold conjugate containing region 29. The device is placed in liquid and liquid flow is as described with reference to the embodiment of Figure 2a. However, since the capture line 28b is above the barrier 26 in this embodiment, the sample and gold conjugate incubate prior to flowing over the capture line 28b. As before, after a predetermined time the barrier 26 switches causing the gold-analyte complex to flow over the capture line 28b. In this embodiment, it is not necessary for methyl cellulose to be applied to the second or third channels 23,24.

Referring to Figure 2c, an assay device closely resembling that of Figure 2b is shown, the only structural difference being that an additional impervious line 21a is provided partially across the upper end of the first and second channels 22,23 where they merge into the common enlarged region 24a. The additional impervious line 21 a is perpendicular to and touches the end of the impervious line 21 defining the first and second channels 22,23 (i.e. said impervious lines 21,21a form a "T" shape). In this particular embodiment the switchable barrier 26 is coumarone resin (plotted using a 0.7 mm pen at 4cm/s) and the surfactant is 2.5% ODG (plotted using a 0.7 mm pen at 5 cm/s). In addition, the surface of the nitrocellulose is covered with a protective transparent film (0.127 mm thick type 8192 film coated on one side with acrylic adhesive AS-110, Adhesives Research Ireland Ltd., Limerick, Ireland).

In use, it was found necessary to prewash and dry the membrane prior to device fabrication to prevent premature opening of the barrier when the combination of coumarone resin and ODG are used. This is thought to be due to unspecified surfactants contained in the supplied membrane. This precaution applies equally to any device where this particular surfactant/barrier combination is used and where liquid moves a relatively long distance to a switchable barrier and remains there for some time prior to switching.

Subsequent application of liquid and fluid flow is as described with reference to Figure 2b, except it was found that the provision of the additional impervious line 21 a straightened the liquid front as it approached the surfactant line 27 and so promoted more even switching of the barrier 26.

The effectiveness of the coumarone resin barrier and ODG surfactant combination was separately evaluated with water, urine, plasma and serum.

### EXAMPLE 3

Referring to Figure 3, a polyester backed 8 µm nitrocellulose membrane 30 is divided into channels by impervious pen lines 31 as described for Example 2a. A vertical first channel 32 leads to left and right inner and outer channels 33l,33r;34l,34r at its upper end, its lower end defining the base of the device. Each outer channel 34l,34r extends around the periphery of the membrane 30 and has an enlarged end region 35l,35r remote from the vertical first channel 31. The right outer channel 34r has a convoluted region 36 so that the length of the right outer channel 34r is greater than that of the left outer channel 341. The left and right inner channels 33l,33r are short in relation to the outer channels 34l,34r and lead into the leftmost and rightmost ends respectively of a horizontal reaction channel 37. The right inner channel 33r also leads into a first overflow channel 38 which is otherwise closed. The reaction channel 37 is provided with a capture line 39 (containing immobilised antibody to analyte of interest) at its midpoint and a gold conjugate containing region 40 spaced to the left of the capture line 39. In other embodiments, the gold conjugate is replaced by different visualising agents.

The enlarged end regions 35l,35r of the left and right outer channels 34l,34r are separated from second and third overflow regions 411,41 r respectively by a first "Type 1" barrier 42l,42r (prepared by centrifuging the silver paint contained in pen type 8700 at 750 g for 20 minutes, diluting the supernatant therefrom with 5% white spirit and plotting with a 0.5 mm pen at 1 cm/s) and from the left and rightmost ends respectively of the reaction channel 37 by a second similar barrier 43l,43r perpendicular to the first barrier 42l,42r. The first and second barriers 42l,42r;43l,43r were dried at 22°C for 2 minutes. Each enlarged end region 35l,35r contains a line of surfactant 44l,44r (2.5% ODG plotted using a 0.7 pen at 5 cm/s, and dried at 35° C for 20 minutes) spaced from and parallel to the respective first and second barriers 42l,43l;42r,43r.

In use, sample being analysed for an analyte of interest is applied to the gold conjugate containing region 40 and the vertical channel 32 of the device is placed into water (or other liquid) which wicks up the vertical channel 32 and into the left and right inner and outer channels 33l,33r,34l,34r. From the left and right inner channels 33l,33r, water flows into the left and right end respectively of the reaction channel 37. Flow from the left end passes through the gold conjugate containing region 40 and washes gold-analyte complex (and gold-conjugate and unreacted sample) towards the capture line 39. Since part of the flow from the right inner channel 33r is diverted into the first overflow channel 38, the flow into the right end of the reaction channel 37 is less than into the left end. Thus, there is net flow in the reaction channel 37 from left to right and the sample is carried across the capture line 39 (left to right) for a first time, where it reacts with the immobilised antibodies. When the first overflow channel 37 is filled, flow in channel 38 substantially stops.

Simultaneously, water continues to flow along the relatively longer outer channels 34l,34r, but because the left outer channel 341 is shorter than the right outer channel 34r, water arrives at the enlarged end region 35l of the left outer channel 341 before the enlarged end region 35r of the right outer channel 34r. A part-circular water front flows into the surfactant line 44l and water now containing surfactant flows simultaneously to the first and second barriers 42l,43l. The surfactant switches the barriers 42l,43l so that liquid can flow therethrough. Thus, the enlarged end region 351 of the left outer channel 341 is in communication with both the left end of the reaction channel 37 and the second overflow channel 41l. Since the second overflow channel 41l is dry, liquid is drawn into the second overflow channel 41l from the enlarged end region 35l of the left outer channel 341 and the left end of the reaction channel 37. This causes right to left flow in the reaction channel 37 and the sample/gold conjugate passes through the capture line 39 (right to left) for a second time.

Meanwhile, water continues to flow in the right outer channel 34r and into its enlarged end region 35r. A part-circular water front flows into the surfactant line 44r and water now containing surfactant flows simultaneously to the first and second barriers 42r,43r. The surfactant switches the barriers 42r,43r so that liquid can flow therethrough. Thus, the enlarged end region 35r of the right outer channel 34r is in communication with both the right end of the reaction channel 37 and the third overflow channel 41 r. Since the third overflow channel 41 r is dry, liquid is drawn into the third overflow channel 41 r from the enlarged end region 35r of the right outer channel 34r and the right end of the reaction channel 37. This causes left to right flow in the reaction channel 37 and the sample/gold conjugate passes through the capture line 39 (left to right) for a third time. This last movement along the reaction channel 37 moves the gold clear of the capture line 39 and provides a wash step so that the capture line 39 can easily be seen.

It will be understood that the distances moved by the sample/gold across the capture line 39 is determined by the volume of the first, second and third overflow regions 38,41l,41r. Such a device offers improved sensitivity when using a small sample volume by allowing sample and reagents to pass several times over the capture line 39.

### EXAMPLE 4

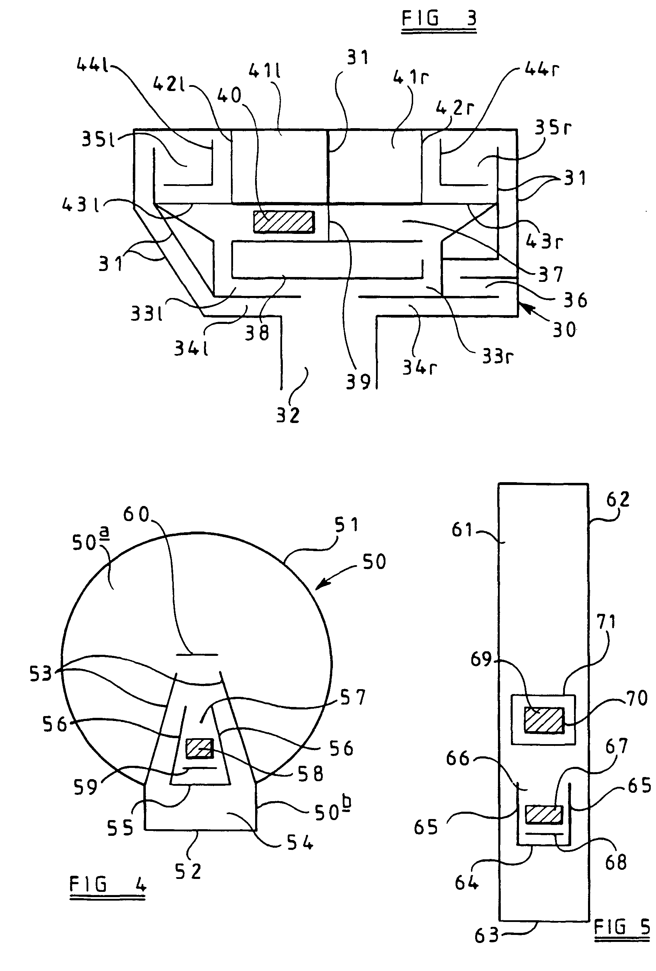

Referring to Figure 4, a polyester backed 8 µm nitrocellulose membrane 50 is divided into channels by impervious pen lines of neat Lumocolor ink as described for Example 2a. The membrane 50 has a substantially circular portion 50a and a rectangular base 50b. The periphery of the membrane 50 is marked with an impervious line 51 except for a bottom edge 52 of the base 50b which serves as a liquid inlet. An impervious line 53 extends generally towards the centre of the circular portion 50a from each side of the base 50b where the base 50b intersects the circular portion 50a to define an open-ended tapering first channel 54. A switchable barrier 55 extends partly across the first channel 54 at the same radius as the impervious line 51 marking the periphery of the membrane 50. A further impervious line 56 extends generally towards the centre of the circular portion 50a from each end of the switchable barrier 55 to define an open-ended tapering second channel 57 wholly within the first channel 54. Within the second channel 57 is disposed a gold conjugate (or other visualising agent) containing region 58 spaced above a line of surfactant 59 (1.25% ODG plotted using a 0.5 pen at 5 cm/s, and dried at 35° C for 10 minutes). A capture line of immobilised antibody 60 is positioned above the first channel 54 and is parallel to and above the switchable barrier 55.

In use, the base 50b of the membrane 50 is placed in a liquid sample. Liquid wicks into the first channel 54 and around the second channel 57, upward flow into the second channel 57 being prevented by the switchable barrier 55. Liquid then flows down into the second channel 57 (moving the gold conjugate and surfactant line towards the switchable barrier 55) and upwards out of the first channel 54 where it forms a part-circular front. The front passes through the capture line 60 and outwards towards the periphery.

The downwardly flowing sample together with the gold conjugate in the second channel 57 wicks into the surfactant line 59 and continues until it contacts the switchable barrier 55. The surfactant acts to convert the capillaries of the barrier 55 from non-wetting to wetting in order for liquid to flow therethrough. However, flow through the barrier 55 does not occur immediately.

Since the switchable barrier 55 is located at about the same radius as the periphery of the membrane 50, outward flow reaches the periphery at about the same time as downward flow in the second channel 57 reaches the switchable barrier 55. When the saturation at the switchable barrier 55 has reached a critical value (dependent upon the barrier/surfactant combination) the barrier 55 becomes permeable. Outward flow towards the periphery continues drawing liquid through the second (and first) channels 57,54, thereby causing gold conjugate to flow over the capture line 60 until total saturation occurs. Any analyte present in the sample will be visualised at the capture site 60, with any unbound material being washed clear.

Switching of different barriers was investigated using water as the sample and the results were as follows:-
1 A "Type 2" barrier (prepared by diluting 1 ml of type 1 barrier material with 250 µl of white spirit and 250 µl of ethanol and plotted using a 0.5 mm pen at 5 cm/s) opened at 3 minutes 50 seconds.
2 White paint barrier plotted using a 0.7 mm pen at 1 cm/s opened at 1 minute 10 seconds (before liquid reached the periphery).
3 1 part Lumocolor in 4 parts 1-propanol barrier plotted using a 0.5 mm pen at 5 cm/s opened at 3 minutes 30 seconds.

This embodiment provides a means of first delivering a large volume of sample through an antibody capture line followed by a visualising agent.

### EXAMPLE 5

Referring to Figure 5, a rectangular polyester backed 8 µm nitrocellulose membrane strip 61 is marked around its periphery by an impervious pen line 62 of neat Lumocolor ink as described for Example 2a except for a short bottom edge 63 which serves as a liquid inlet. Parallel to the bottom edge 63, a first Type 2 material switchable barrier 64 (plotted using a 0.5mm pen at 5 cm/s) extends partly across the strip 61. An impervious line 65 extends perpendicularly from each end of the first barrier 64 away from the bottom edge 63 to define a channel 66 having a closed lower end and an open upper end. Within the channel 66 is a colorimetric reagent containing region 67 spaced above a line of surfactant 68 (2.5% ODG plotted using a 0.7mm pen at 5 cm/s, and dried at 35° C for 10 minutes). Above the channel 66, a capture region 69 is defined within a rectangular line of second Type 2 switchable barrier 70. The capture region 69 contains immobilised antibody and an enzyme conjugate. In this embodiment, the colorimetric reagent is one which interacts with the enzyme conjugate and not the analyte itself. A rectangular line of surfactant 71 (also 2.5% ODG) surrounds the capture region 69 and is spaced from the second barrier 71.

In use, about 3 µl of sample to be analysed for the analyte of interest is applied onto the capture region 69 eg. By pipetting. The sample is contained within the capture region 69 by the second switchable barrier 70. The enzyme conjugate is solubilised and if the analyte of interest is present in the sample it becomes bound to the immobilised antibody and the enzyme conjugate. The bottom edge 63 of the membrane is then placed in water (or other suitable diluent). Water flows along the strip 61 around the channel 66 but cannot flow into the channel 66 from below because of the first barrier 64. Flow continues along the strip 61 towards the rectangular line of surfactant 71. When surfactant reaches the second barrier 70, the second barrier 70 is switched to allow water to flow therethrough. The capture region 69 is than totally open to flow and sample and any excess enzyme conjugate is washed upwards away from the capture region 69.

During this time flow enters the channel 66 from above and the colorimetric reagent and line of surfactant 68 move towards the first barrier 64. When the barrier 64 is switched, flow in the channel 66 is reversed and the colorimetric reagent moves over the capture region 69. If the analyte of interest is present, the colorimetric substrate reacts with the enzyme conjugate to produce visible colour at the capture region 69. In this embodiment, the relative dimensions of the device and positioning of the various elements of the device are such that the device becomes totally saturated at substantially the same time as the colorimetric reagent reaches the capture region, so that the colorimetric reagent remains at the capture region.

The device is also designed so that the first barrier 64 is switched after the second barrier 70 to ensure that the colorimetric reagent passes into the capture region 69 and not around it.

This embodiment provide a means of analysing sample using a multi-reagent assay. It will be noted that excess conjugate is automatically washed away from the capture region 69 prior to arrival of colorimetric reagent at the capture region 69. The device is particularly suited for screening assays and offers an automatic non-instrumental alternative to microtitration plate assays.

Example 5 describes a device having a single capture region 69, but in a modification, several capture regions are provided so that a number of samples can be analysed in a single assay.

### EXAMPLE 6

Referring to Figure 6a, a rectangular 12 µm nominal pore size unsupported nitrocellulose membrane strip 80 (type AE 100, Schleicher & Schuell, Dassel, Germany) is marked with impervious pen lines 81 of neat Lumocolor ink parallel to and marginally spaced from its long side edges 82. Parallel to a first short side edge 83, a first Type 2 switchable barrier 84 extends partly across the strip 80 (plotted using a 0.7 mm pen at 1 cm/s and dried at 22° C for 2 minutes). An impervious line 85 extends perpendicularly from each end of the first barrier 84 away from the first side edge 83 towards a second short side edge 86 to define a channel 87 having a closed first end and an open second end. Within the channel 87 is a gold conjugate containing region 88 spaced to the left of a line of surfactant 89 (10% ODG plotted using a 0.7 pen at 5 cm/s). Disposed between the second short side edge 86 and the channel 87, a capture region 90 is defined within an elliptical ring of a second Type 2 switchable barrier 91. The capture region 90 contains immobilised antibody. An elliptical ring of surfactant 92 (also 10% ODG) surrounds the capture region 90 and is spaced from the second barrier 91.

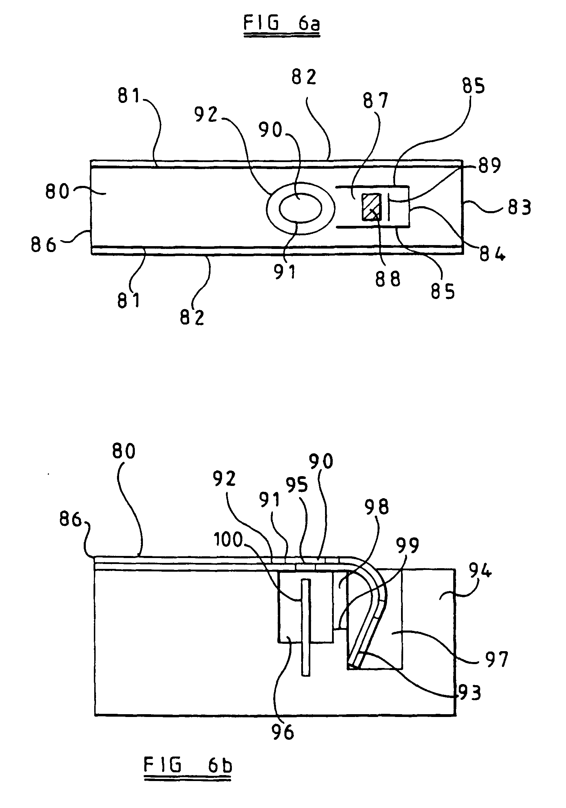

Referring to Figure 6b, the unsupported membrane strip 80 of Figure 6a is adhered to a polyester backing 93 (0.025 mm thick type 7759 film coated on one side with acrylic adhesive AS-110, Adhesives Research Ireland Ltd, Limerick, Ireland) and mounted to a housing 94. It should be noted that for unsupported membrane material, one face tends to have larger pores than the other face. Under low liquid pressure loads, liquid does not flow easily through the membrane from the large pore face to the small pore face. The polyester backing 93 is adhered to the large pore face of the membrane strip 80 (identified by being less shiny than the small pore face). The backing 93 is provided with an aperture 95 therethrough which is aligned with the capture region 90 of the membrane strip 80.

The housing 94 is machined from Perspex acrylic sheet (ICI, Darwen, UK) and is generally cuboidal. First and second troughs 96,97 extend partly across the width of the housing 94. The second trough 97 is deeper than the first trough 96 and is in communication therewith by virtue of a flow passage 98. The base of the flow passage 98 is higher than the base of the first trough 96 so that a lip 99 is defined between the first and second troughs 96,97. Within the first trough 96 is embedded a peg 100 which projects vertically. The polyester backed membrane is mounted flat on an upper surface of the housing 94 in such a position that the capture region 90 is directly above the peg 100 in the first trough 96. An end portion of the membrane is bent downwardly so that its first short side edge 83 is in contact with the base of the second trough 97.

In use, sample is applied to the capture region 90 using a pipette (in an alternative embodiment, not shown, a funnel is positioned above the capture region 90 and loaded with a pre-determined volume of sample which is automatically applied to the membrane at a desired rate) and is constrained from flowing into the surrounding membrane by the second barrier 91. Sample quickly flows vertically through the thickness of the membrane 80 from the small pore face of the membrane 80 to the large pore face and through the aperture 95 in the polyester film 93. If the analyte of interest is present it will become bound to the immobilised antibody capture region 90. Pin 100 helps to guide flow down into the first trough 96. When sufficient sample is applied, sample flows over the lip 99 into the second trough 97 and wets the first short side edge 83 of the membrane 80. Sample flow is then as described with reference to Example 5 resulting in (i): washing of unbound sample from the capture region 90, (ii) flow of gold conjugate over the capture region 90 and (iii) flow of excess gold conjugate away from the capture region 90.

This embodiment allows a large volume of sample to be analysed, important if the analyte of interest is present in low concentrations. Furthermore, the sample volume, after passing vertically down through a relatively small capture site is directed to the end of the strip and flows back through the capture site horizontally. It is particularly suited for detecting bacteria (eg. Coliforme) in diluted samples as is often required in environmental tests.

In a modification of the above embodiment, the troughs 96,97 contain porous or absorbent material to absorb excess liquid and prevent it from escaping from the housing 94 after use. It will be understood that the purpose of the troughs 96,97 in the housing 94 is to enable a predetermined volume of sample to be applied to the capture region 90 before flow is initiated along the membrane 80. The same effect may be achieved by other methods. For example, in another modification, sample flowing through the membrane passes into porous material, or a capillary channel which is in abutment with the first short edge of the membrane (see Example 8). Suitable porous materials include glass fibre fleece, cellulose fibre fleece, polymeric sponge-like material and poly(isobutylene-co-maleic acid) sodium salt, with good absorbent properties.

### EXAMPLE 7

Referring to Figure 7a, a rectangular 8 µm nominal pore size unsupported nitrocellulose membrane strip 100 (type AE99, Schleicher and Schuell) with the small pores uppermost, is marked with an impervious pattern of pen lines using neat Lumocolor ink (plotted using a 0.7 mm pen at 3 cm/s and dried at 22°C for 2 minutes) to define an inner channel 102 between a pair of outer channels 104, all of which channels 102,104 extend from a first (lower) common region 106 to a second (upper) common region 108 (references to "upper" and "lower" are in relation to the orientation depicted in Figure 7a). Specifically, the inner channel 102 is defined by a pair of spaced apart parallel impervious lines 110 which are joined at their lower ends by a first line of coumarone resin 112 (prepared as described in Table 10) serving as a switchable barrier (plotted using a 0.7 mm pen at 3 cm/s and dried at 22°C for 2 minutes). An additional impervious line 114 extends perpendicularly to and is spaced above the first pair of parallel impervious lines, with the ends 114a of the additional impervious line 114 extending a short distance vertically downwardly. The portion of the additional impervious line 114 extending perpendicularly to the parallel impervious lines 110 being longer than the width of the inner channel 102. The ends 114a of the additional impervious line 114 and the upper ends of the parallel impervious lines 110 are connected by a respective one of a pair of additional coumarone resin (switchable barrier) lines 116. It will therefore be understood that in the configuration described, the inner channel 102 is completely enclosed.

The outer channels 104 are defined by further impervious lines 118, as well as the parallel impervious lines 110 and the additional coumarone resin lines 116 described above. The lower ends of the outer channels 104 are mutually parallel, with the upper ends mutually diverging towards the edges of the membrane strip 100.

A capture region 120 containing immobilised antibody is provided towards the upper end of the inner channel 102 (between the parallel impervious lines 110), with a gold conjugate region 122 being provided below the capture region 120 also in the inner channel 102. A line of surfactant 124 (10% solution of ODG plotted using a 0.7 mm pen at 5 cm/s) extends downwardly along one of the outer channels 104, underneath the inner channel 102 and upwardly along the other outer channel 104. In the embodiment shown, a further coumarone switchable barrier 126 is provided immediately below the first coumarone resin line 112 and a further line of 10% ODG surfactant 128 is provided below the first line of 10% ODG surfactant underneath the inner channel 102. These further lines of coumarone resin barrier 126 and ODG surfactant 128 are optional, but have been found to promote more reliable switching of the coumarone resin barrier 112 in use.

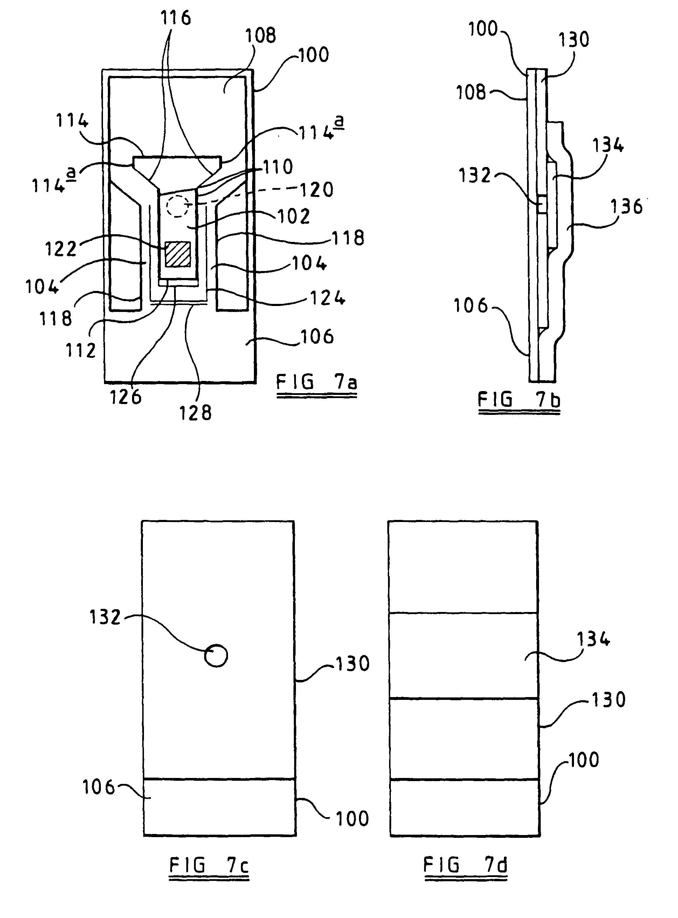

Referring to Figure 7b, adhered beneath (to the right in Figure 7b) the nitrocellulose membrane strip 100 is a polyester backing 130 (0.254 mm thick type 8565 film coated on one side with acrylic adhesive AS-110, Adhesives Research). The backing 130 is provided with an aperture 132 (the edge of the aperture 132 being coated with 10% ODG solution applied from the direction of the non-adhesive side) and is shorter in length than the nitrocellulose membrane strip 100. The backing 130 is adhered to the membrane strip 100 such that the aperture 132 is directly beneath the capture region 120 containing the immobilised antibody and so that the first (lower) common region 106 of the membrane strip 100 extends below the backing 130 (Figure 7c). A strip of silk 134 (or other think fabric such as synthetic fabric type PE33HC (ZBF, Zurich) is adhered across the backing 130 so as to cover the aperture 132. This is achieved by masking the region of the aperture 132 and applying a light spray of adhesive (Spraymount adhesive, 3M United Kingdom PLC., UK) to the surface of the backing 130 (Figure 7d).

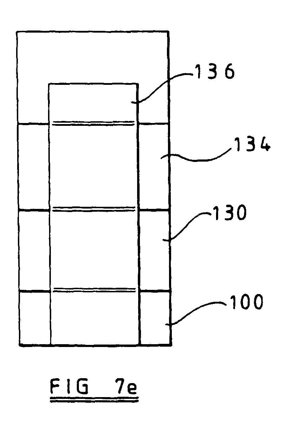

It should be noted that if the synthetic fabric is used, this is first impregnated with 10% ODG solution and dried before being used. A strip of porous material 136 , in this case Vileda Sunsplash [TM] (Freudenberg Household Products LP, Rochdale) is adhered to the backing 130 (above and below the silk strip 134, to the silk strip 134 itself and the lower end of the nitrocellulose membrane strip 100 - see Figure 7e). This is achieved by applying a further light spray of Spraymount adhesive to the fabric side of the device and gently compressing the Vileda Sunsplash porous material 136 onto the device. It should be noted that the porous material is only in direct contact with the membrane at the lower end of the latter. The strip of porous material 136 is narrower than the membrane strip 100 and does not extend as far as the side edges of the latter (Figure 7e).

In use, the device is supported with the membrane strip 100 in a horizontal position and sample applied to the capture region 120 using a pipette. Although it will be appreciated that in alternative embodiments, a sample application well may be incorporated to a housing for the device and positioned over the capture region 120 (not shown). Sample is constrained from flowing out of the inner channel 102 by the impervious lines 110, 114 and the coumarone resin lines 112,116. Thus, as more sample is applied, it flows vertically through the thickness of the membrane strip 100 and through the aperture 132 in the polyester backing 130. If the analyte of interest is present, it will become bound in the immobilised antibody capture region 120. Sample flowing through the aperture 132 in the polyester backing 130 flows into the silk 134 and then into the porous material 136. Sample continues to flow along the porous material 136 and, because it is in direct contact with the membrane strip 100, back into the membrane strip 100 into the first (lower) common region 106 of the latter. The capillarity of the porous material 136 is higher than that of the aperture 132, and the volume of the porous material 136 is chosen such that after the entire sample has been applied, any free liquid in the inner channel 102 is absorbed into the porous material 136 and the aperture 132 is clear of sample. As the sample flows from the first (lower) common region 106 towards the second (upper) common region 108, the ODG surfactant lines 124,128 are moved towards the coumarone resin barriers 112,126 at the lower end of the inner channel 102 which are consequently opened by the action of the ODG surfactant. Sample continues along the outer channels 104, but does not flow through the inner channel 102 since the additional coumarone resin barriers 116 have not yet opened (flow of sample through a channel requires an outlet in that channel). When the ODG initially present in the outer channels 104 is moved by sample flow into the additional coumarone resin barriers 116, the latter are switched thereby providing an outlet from the inner channel 102. As a result, sample flows along the inner channel 102 so as to wash the gold conjugate over the analyte capture region 120 where it becomes bound if the analyte of interest is present. Further flow causes unbound gold conjugate to clear the capture region 120 in order for the result to be clearly observed.

It will be understood that sample cannot flow from the porous material 136 upwardly through the aperture 132 thereby causing flow distortion in the inner channel 102, because the aperture 132 is already clear of sample. Futhermore, the silk 134 prevents fibres projecting from the surface of the porous material 136 from forming a fluid pathway back through the aperture onto the membrane strip 100. Thus, in alternative embodiments (not shown) the Vileda Sunsplash material is replaced by an absorbent material without projecting surface fibres thereby moving the necessity for the silk 134 between the polyester backing 130 and the porous material 136.

As with Example 6, this embodiment allows a large volume of sample to be analysed, with most of the sample flowing through the capture region first vertically downwards and subsequently horizontally through the capture region.

## Claims

1. A liquid-flow control device comprising:-
(i) a liquid flow path, defined by interstices of a porous medium
(ii) a liquid flow barrier at a location in the flow path, the liquid flow barrier being a non-wetting region in the flow path, and
(iii) barrier release means impregnated in the porous medium in the flow path, the barrier release means being a wetting agent capable of wetting said liquid flow barrier and which, in use, is moveable by liquid flowing in the flow path into contact with the fluid flow barrier so as to permit liquid flow through said location.

2. A device as claimed in claim 1, wherein said liquid is an aqueous liquid.

3. A device as claimed in claim 1 or 2, wherein the liquid flow barrier is a hydrophobic material and the release means is a surfactant.

4. A device as claimed in claim 3, wherein the liquid flow barrier is selected from the group consisting of waxes, resins, synthetic polymers, inks and paints.

5. A device as claimed in claim 3 or 4, wherein the release means is selected from the group consisting of octyl-β-D-glucopyranoside, dioctyl sulfosuccinate sodium salt, polyoxyethylene (23) dodecyl ether and polyoxyethylene (20) sorbitan monolaurate.

6. A device as claimed in any preceding claim, wherein the liquid flow barrier is impregnated into and immobilised on the porous medium.

7. A device as claimed in any preceding claim, wherein the device is constructed so that, in use, there is at least one region in the flow path where the direction of liquid flow is different after liquid flow through said location is initiated compared with the direction of liquid flow in that region before liquid flow through said location is initiated.

8. An assay device for an analyte comprising:-
(i) a flow path for a liquid defined by interstices in a porous medium,
(ii) at least one liquid flow barrier on the porous medium at a location in the flow path, said barrier being a non-wetting region in the flow path
(iii) at least one barrier release means which is a wetting agent capable of wetting said liquid flow barrier, is impregnated into the porous medium, is soluble in the liquid, and which, in use, is moveable by liquid flowing in the flow path into contact with the flow barrier so as to permit liquid flow through said location, and
(iv) an analyte capture region in the flow path.

9. A device as claimed in claim 8, wherein the device is provided with a visualising agent application zone adapted to receive a visualising agent.

10. A device as claimed in claim 8, wherein an analyte visualising agent is provided at a zone in the flow path, said visualising agent being capable of interacting with the analyte to indicate the presence of the analyte.

11. A device as claimed in claim 9, wherein the visualising agent is immobilised in the capture region and also serves to immobilise the analyte in the capture region.

12. A device as claimed in claim 9, wherein the analyte capture region includes an immobilised analyte binding substance which serves to immobilise analyte in the capture region and the visualising agent is upstream of the capture region so that in use, it flows through the capture region.

13. A device as claimed in any one of claims 8 to 12, wherein a sample application zone is provided.

14. A device as claimed in claim 13, wherein the sample application zone corresponds to the analyte capture region or the visualising agent application zone of claim 9.

15. A device as claimed in claim 13 or 14, wherein the sample application zone is surrounded by an additional flow barrier and an associated barrier release means.

16. A device as claimed in any one of claims 8 to 14, wherein the sample to be analysed is included in the liquid which flows, in use, along the flow path.

17. A device as claimed in any one of claims 8 to 16, wherein at least two barriers and at least two barrier release means are provided and arranged so that, in use, some liquid flows over the analyte capture region at least twice.

## Patentansprüche

1. Flüssigkeitsströmungssteuervorrichtung, umfassend:
(i) einen Flüssigkeitsströmungsweg, der durch Zwischenräume eines porösen Mediums definiert ist;
(ii) eine Flüssigkeitsströmungssperre an einem Ort im Strömungsweg, wobei die Strömungssperre eine nichtnetzende Region im Strömungsweg ist; und
(iii) Sperrenaufhebungsmittel, die in das poröse Medium im Strömungsweg getränkt sind, wobei die Sperrenaufhebungsmittel jeweils ein Benetzungsmittel sind, mit dem die Benetzung der Flüssigkeitsströmungssperre möglich ist und die, im Einsatz, durch die Flüssigkeit, die im Strömungsweg strömt und mit der Flüssigkeitsströmungssperre in Kontakt kommt, beweglich sind, damit die Flüssigkeitsströmung durch den Ort möglich ist.

2. Vorrichtung nach Anspruch 1, wobei die Flüssigkeit eine wässrige Flüssigkeit ist.

3. Vorrichtung nach Anspruch 1 oder 2, wobei die Flüssigkeitsströmungssperre ein hydrophobes Material und das Aufhebungsmittel ein grenzflächenaktiver Stoff ist.

4. Vorrichtung nach Anspruch 3, wobei die Flüssigkeitsströmungssperre ausgewählt wird aus der Gruppe, bestehend aus Wachsen, Harzen, synthetischen Polymeren, Druckfarben und Lacken.

5. Vorrichtung nach Anspruch 3 oder 4, wobei die Aufhebungsmittel ausgewählt werden aus der Gruppe, bestehend aus Octyl-β-D-glucopyranosid, Dioctylsulfosuccinat-Natriumsalz, Polyoxyethylen-(23)-Dodecylether und Polyoxyethylen-(20)-Sorbitanmonolaurat.

6. Vorrichtung nach einem der vorangehenden Ansprüche, wobei die Flüssigkeitsströmungssperre in das poröse Medium getränkt und an demselben immobilisiert ist.

7. Vorrichtung nach einem der vorangehenden Ansprüche, wobei die Vorrichtung so ausgeführt ist, dass, im Einsatz, mindestens eine Region im Strömungsweg vorhanden ist, wo die Richtung des Flüssigkeitsstromes nachdem die Flüssigkeitsströmung durch den Ort initiiert wurde im Vergleich zu der Richtung der Flüssigkeitsströmung in der Region, bevor die Flüssigkeitsströmung durch diesen Ort initiiert wurde, anders ist.

8. Assayvorrichtung für einen Analyten, umfassend:
(i) einen Strömungsweg für eine Flüssigkeit, der durch Zwischenräume in einem porösen Medium definiert ist;
(ii) mindestens eine Flüssigkeitsströmungssperre am porösen Medium an einem Ort im Strömungsweg, wobei die Sperre eine nichtnetzende Region im Strömungsweg ist;
(iii) mindestens ein Sperrenaufhebungsmittel, das ein Benetzungsmittel ist, mit dem die Benetzung der Flüssigkeitsströmungssperre möglich ist, das in das poröse Medium getränkt ist, das in der Flüssigkeit löslich ist, und das, im Einsatz, durch die Flüssigkeit, die im Strömungsweg strömt und mit der Strömungssperre in Kontakt kommt, beweglich ist, damit die Flüssigkeitsströmung durch den Ort möglich ist; und
(iv) eine Analytaufnahmeregion im Strömungsweg.

9. Vorrichtung nach Anspruch 8, wobei die Vorrichtung mit einer Visualisierungsagensauftragszone bereitgestellt wird, die zur Aufnahme eines Visualisierungsagens ausgelegt ist.

10. Vorrichtung nach Anspruch 8, wobei ein Analytvisualisierungsagens an einer Zone im Strömungsweg bereitgestellt wird und das Visualisierungsagens mit dem Analyten wechselwirken kann, um das Vorhandensein des Analyten anzuzeigen.

11. Vorrichtung nach Anspruch 9, wobei das Visualisierungsagens in der Aufnahmeregion immobilisiert ist und außerdem dazu dient, den Analyten in der Aufnahmeregion zu immobilisieren.

12. Vorrichtung nach Anspruch 9, wobei die Analytaufnahmeregion eine immobilisierte Analytbindungssubstanz umfasst, die zum Immobilisieren des Analyten in der Aufnahmeregion dient, und das Visualisierungsagens sich so stromaufwärts der Aufnahmeregion befindet, dass es, im Einsatz, durch die Aufnahmeregion strömt.

13. Vorrichtung nach einem der Ansprüche 8 bis 12, wobei eine Probenauftragszone bereitgestellt wird.

14. Vorrichtung nach Anspruch 13, wobei die Probenauftragszone der Analytaufnahmeregion oder der Visualisierungsagensauftragszone von Anspruch 9 entspricht.

15. Vorrichtung nach Anspruch 13 oder 14, wobei die Probenauftragszone von einer zusätzlichen Strömungssperre und von einem zugeordneten Sperrenaufhebungsmittel umgeben ist.

16. Vorrichtung nach einem der Ansprüche 8 bis 14, wobei die zu analysierende Probe in der Flüssigkeit enthalten ist, die, im Einsatz, längs des Strömungsweges strömt.

17. Vorrichtung nach einem der Ansprüche 8 bis 16, wobei mindestens zwei Sperren und mindestens zwei Sperrenaufhebungsmittel bereitgestellt und so angeordnet werden, dass, im Einsatz, mindestens zweimal etwas Flüssigkeit über die Analytaufnahmeregion strömt.

## Revendications

1. Dispositif de contrôle de l'écoulement d'un liquide, comprenant
(i) une trajectoire d'écoulement du liquide, définie par des interstices d'un agent poreux
(ii) une barrière de l'écoulement du liquide agencée au niveau d'un emplacement dans la trajectoire d'écoulement, la barrière de l'écoulement du liquide étant constituée par une région sans mouillage dans la trajectoire d'écoulement, et
(iii) un moyen de dégagement de la barrière imprégné dans l'agent poreux dans la trajectoire d'écoulement, le moyen de dégagement de la barrière étant un agent mouillant capable de mouiller ladite barrière d'écoulement du liquide, et pouvant être déplacé en service par l'écoulement du liquide dans la trajectoire d'écoulement, en contact avec la barrière d'écoulement du liquide, de sorte à permettre l'écoulement du liquide à travers ledit emplacement.

2. Dispositif selon la revendication 1, dans lequel ledit liquide est un liquide aqueux.

3. Dispositif selon les revendications 1 ou 2, dans lequel la barrière d'écoulement du liquide est constituée par un matériau hydrophobe, le moyen de dégagement étant constitué par un agent de surface.

4. Dispositif selon la revendication 3, dans lequel la barrière d'écoulement du liquide est sélectionnée dans le groupe constitué de cires, de résines, de polymères synthétiques, d'encres et de peintures.

5. Dispositif selon les revendications 3 ou 4, dans lequel le moyen de dégagement est sélectionné dans le groupe constitué de octyl-β-D-glucopyranoside, de sel de sodium de sulfosuccinate de dioctyle, d'un éther de polyoxyéthylène (23) dodécyle et de monolaurate de sorbitan polyoxyéthyléné (20).

6. Dispositif selon l'une quelconque des revendications précédentes, dans lequel la barrière d'écoulement du liquide est imprégnée dans et immobilisé sur l'agent poreux.

7. Dispositif selon l'une quelconque des revendications précédentes, dans lequel le dispositif est construit de sorte qu'en service, il existe au moins une région dans la trajectoire d'écoulement dans laquelle la direction de l'écoulement du liquide est différente après l'initiation de l'écoulement du liquide à travers ledit emplacement de la direction d'écoulement du liquide dans cette région avant l'initialisation de l'écoulement du liquide à travers ledit emplacement.

8. Dispositif d'essai pour une substance à analyser, comprenant:
(i) une trajectoire d'écoulement pour un liquide définie par des interstices dans un agent poreux;
(ii) au moins une barrière de l'écoulement du liquide sur l'agent poreux agencée au niveau d'un emplacement dans la trajectoire d'écoulement, la barrière de l'écoulement du liquide étant constituée par une région sans mouillage dans la trajectoire d'écoulement, et
(iii) au moins un moyen de dégagement de la barrière constitué par un agent mouillant capable de mouiller ladite barrière d'écoulement du liquide, imprégné dans l'agent poreux, soluble dans le liquide et pouvant être déplacé en service par le l'écoulement du liquide dans la trajectoire d'écoulement, en contact avec la barrière d'écoulement du liquide, de sorte à permettre l'écoulement du liquide à travers ledit emplacement, et
(iv) une région de capture de la substance à analyser dans la trajectoire d'écoulement.

9. Dispositif selon la revendication 8, dans lequel le dispositif comporte une zone d'application d'un agent de visualisation destinée à recevoir un agent de visualisation.

10. Dispositif selon la revendication 8, dans lequel un agent de visualisation d'une substance à analyser est agencé au niveau d'une zone dans la trajectoire d'écoulement, ledit agent de visualisation étant capable de coopérer avec la substance à analyser pour indiquer la présence de la substance à analyser.

11. Dispositif selon la revendication 9, dans lequel l'agent de visualisation est immobilisé dans la région de capture et sert aussi à immobiliser la substance à analyser dans la région de capture.

12. Dispositif selon la revendication 9, dans lequel la région de capture de la substance à analyser englobe une substance de liaison de la substance à analyser immobilisée servant à immobiliser la substance à analyser dans la région de capture, l'agent de visualisation étant agencé en amont de la région de capture, de sorte à s'écouler en service à travers la région de capture.

13. Dispositif selon l'une quelconque des revendications 8 à 12, comportant une zone d'application d'un échantillon.

14. Dispositif selon la revendication 13, dans lequel la zone d'application de l'échantillon correspond à la région de capture de la substance à analyser ou à la zone d'application de l'agent de visualisation de la revendication 9.

15. Dispositif selon les revendications 13 ou 14, dans lequel la zone d'application de l'échantillon est entourée par une barrière d'écoulement additionnelle et d'un moyen de dégagement associé de la barrière.

16. Dispositif selon l'une quelconque des revendications 8 à 14, dans lequel l'échantillon devant être analysé est inclus dans le liquide s'écoulant en service le long de la trajectoire d'écoulement.

17. Dispositif selon l'une quelconque des revendications 8 à 16, comportant au moins deux barrières et au moins deux moyens de dégagement de barrière, agencés de sorte qu'en service une certaine quantité de liquide s'écoule au moins à deux reprises au-dessus de la région de capture de la substance à analyser.
